# FASCICULE DE BREVET EUROPEEN

(11) **EP 3 277 702 B1**
(45) Date de publication et mention de la délivrance du brevet: **06.09.2023**
(21) Numéro de dépôt: 16718427.4
(22) Date de dépôt: 30.03.2016
(51) Int. Cl.: C07K 1/113, C12N 9/24

(54) **STABILISATION DE PROTEINES**
STABILISATION VON PROTEINEN
STABILISATION OF PROTEINS

(30) Priorité: 03.04.2015 FR 1552899
(43) Date de publication de la demande: 07.02.2018
(73) Titulaire: Roquette Frères, 62136 Lestrem (FR)
(72) Inventeur: NONO, Merveille, 62400 Bethune (FR); DESCAMPS, Nicolas, 59262 Sainghin-en-Melantois (FR); SIMON, Denis, 59495 Leffrinckoucke (FR); HAEUSLER, Olaf, 59270 Fletre (FR)
(74) Mandataire: Plasseraud IP
(86) Numéro de dépôt international: PCT/FR2016/050709
(87) Numéro de publication internationale: WO 2016/156734

(56) Documents cités:
- EP-A2- 0 750 843
- WO-A1-91/18091
- RU-C1- 2 470 520
- US-B2- 7 838 055
- XIAOMING LIU ET AL: "Effects of Polyols on the Stability of Whey Proteins in Intermediate-Moisture Food Model Systems", JOURNAL OF AGRICULTURAL AND FOOD CHEMISTRY, vol. 57, no. 6, 25 mars 2009 (2009-03-25), pages 2339-2345, XP055244356, US ISSN: 0021-8561, DOI: 10.1021/jf802789y
- LUCRÈCE NICOUD ET AL: "Effect of polyol sugars on the stabilization of monoclonal antibodies", BIOPHYSICAL CHEMISTRY., vol. 197, 1 février 2015 (2015-02-01), pages 40-46, XP055244053, NL ISSN: 0301-4622, DOI: 10.1016/j.bpc.2014.12.003
- KUNIHIKO GEKKOAND ET AL: "Amino Acid Solubility and Protein Stability in Aqueous Maltitol Solutions", AGRICULTURAL AND BIOLOGICAL CHEMISTRY, vol. 53, no. 1, 1 janvier 1989 (1989-01-01), pages 89-96, XP055244572, JP ISSN: 0002-1369
- SAORI KADOYA ET AL: "Freeze-drying of proteins with glass-forming oligosaccharide-derived sugar alcohols", INTERNATIONAL JOURNAL OF PHARMACEUTICS, ELSEVIER BV, NL, vol. 389, no. 1, 15 janvier 2010 (2010-01-15), pages 107-113, XP028308326, ISSN: 0378-5173, DOI: 10.1016/J.IJPHARM.2010.01.027 [extrait le 2010-01-25]

## Description

La présente invention a pour objet l'utilisation d'une combinaison d'un polyol (i) constitué de 12 atomes de carbone et d'un polyol (ii) constitué de 4 à 6 atomes de carbone, comme stabilisateur de protéines. La présente invention a également pour objet des compositions protéiques thérapeutique ou enzymatique comprenant une telle combinaison. La présente invention a enfin pour objet un procédé de préparation de compositions protéiques mettant en oeuvre une telle combinaison.

### ART ANTERIEUR

Les dernières décennies ont vu s'accélérer le développement des produits thérapeutiques issus des biotechnologies, et en particulier des biomédicaments, qui sont aujourd'hui principalement représentés par les protéines thérapeutiques. Seulement les caractéristiques intrinsèques des protéines sont à l'origine d'une problématique galénique spécifique pour la mise au point de ces médicaments. Leur poids moléculaire élevé associé à leur faible résorption - et donc à une faible biodisponibilité - ainsi que leur sensibilité aux protéases digestives limitent considérablement les modalités possibles pour leur administration et, en particulier, ne permettent pas d'utiliser la voie orale. C'est pour cette raison que les voies injectables sont actuellement privilégiées.

Les protéines se caractérisent également par leur instabilité, leur sensibilité aux variations des conditions environnantes, comme la température ou le pH, et par une interaction potentielle avec les surfaces hydrophobes de leur contenant. Ces paramètres doivent être strictement contrôlés car la dénaturation des protéines et leur modification structurelle risquent de provoquer chez le patient, non seulement une altération de leur efficacité thérapeutique mais aussi des réactions immunitaires néfastes.

Malheureusement la production industrielle de protéines implique des procédés qui peuvent facilement dénaturer ces dernières. Un des principaux défis réside dans leur capacité à résister à ces traitements industriels. De tels traitements sont typiquement la cryogénisation, la lyophilisation, ou encore les traitements thermiques utilisés à des fins de purification ou d'inactivation virale, à des températures proches de celle de la dénaturation des protéines.

La conformation des protéines doit également pouvoir être conservée lors du stockage des protéines. Ceci est d'autant plus vrai que dans le domaine des protéines injectables, l'industrie cherche de plus en plus à remplacer les produits lyophilisés par des produits déjà en solution.

Afin de pallier ces problèmes de stabilité, l'art antérieur fait mention de l'utilisation de différents agents, par exemple le tréhalose, les acides aminés tels que l'arginine et la lysine, les protéines véhicules, les hydrocolloïdes, les tensio-actifs, les cyclodextrines natives ou modifiées, et les solvants.

Le tréhalose est le principal carbohydrate utilisé pour cette application, et a fait l'objet de diverses demandes de brevets (par exemple la demande délivrée sous le numéro US 4,891,319 A). Ce sucre est un disaccharide constitué de 12 atomes de carbone, qui est en pratique généralement utilisé en combinaison avec des protéines véhicule et/ou des hydrocolloïdes.

Le principal problème du tréhalose est qu'il s'agit d'un sucre rare, dont la synthèse est particulièrement complexe et onéreuse. Il est ainsi difficile d'en produire de larges quantités. De plus, il possède un index glycémique tel qu'il est déconseillé chez les patients diabétiques.

Il est du mérite de la Demanderesse d'avoir réussi à développer un additif stabilisateur constitué de carbohydrates, particulièrement utile pour la stabilisation des protéines. Cet additif, bien plus facile d'accès que le tréhalose, consiste en une combinaison d'un premier polyol (i) constitué de 12 atomes de carbone, préférentiellement le maltitol, et d'un second polyol (ii) constitué de 4 à 6 atomes de carbone, préférentiellement le sorbitol, le xylitol et/ou le mannitol.

L'utilisation, pour stabiliser les protéines, de polyols effectivement constitués de 12 atomes de carbone a déjà été décrite dans l'art antérieur. De même, l'utilisation de polyols effectivement constitués de 4 à 6 atomes de carbone a déjà été décrite. Il n'avait cependant jamais été envisagé de les combiner. De manière plus générale, l'art antérieur est extrêmement pauvre pour ce qui concerne les mélanges de carbohydrates. On peut tout de même citer à cet égard :
- l'article Cicerone MT; Tellington A; Trost I; Sokolov A. Substantially improved stability of biological agents in dried form : The role of glassy dynamics in préservation of biopharmaceuticals. BioProc. Int. 1 : 36-47 (2003).
- la demande de brevet WO 98/00530, qui décrit un additif composé d'un disaccharide, d'un dérivé de disaccharide, d'une protéine véhicule et d'un polysaccharide.

Ces publications ne divulguent pas l'utilisation d'une combinaison d'un polyol constitué de 12 atomes de carbone avec un polyol constitué de 4 à 6 atomes de carbone.

Il est intéressant d'ajouter que l'utilisation du maltitol, qui est le polyol en C12 préféré dans la présente invention, n'est que rarement exemplifiée dans l'art antérieur. Les données expérimentales publiées ne révèlent d'ailleurs pas une efficacité particulière de ce dernier. Certaines données vont jusqu'à pointer le manque de performance du maltitol.

### BUTS DE L'INVENTION

Un premier objectif de l'invention consiste en la fourniture d'un agent stabilisateur qui présente des propriétés équivalentes voire améliorées par rapport au tréhalose.

L'invention a en particulier pour objectif de répondre au problème susmentionné en fournissant un agent stabilisateur de protéines qui soit plus facile d'accès que le tréhalose.

L'invention a en particulier pour objectif de répondre aux problèmes susmentionnés en fournissant un agent stabilisateur biosourcé, issu de la technologie des carbohydrates.

### RESUME DE L'INVENTION

La présente invention a pour premier objet l'utilisation d'une combinaison d'un polyol (i) constitué de 12 atomes de carbone et d'un polyol (ii) constitué de 4 à 6 atomes de carbone, comme stabilisateur de protéines.

La présente invention a pour second objet une composition protéique thérapeutique ou enzymatique comprenant une protéine, un polyol (i) constitué de 12 atomes de carbone et un polyol (ii) constitué de 4 à 6 atomes de carbone.

La présente invention a pour troisième objet un procédé de préparation d'une telle composition, comprenant le mélange d'une protéine avec un polyol (i) constitué de 12 atomes de carbone et un polyol (ii) constitué de 4 à 6 atomes de carbone.

### DESCRIPTION DETAILLEE DE L'INVENTION

La Demanderesse a montré qu'une synergie s'opérait entre le polyol (i) constitué de 12 atomes de carbone, en particulier le maltitol, et le polyol (ii) constitué de 4 à 6 atomes de carbone.

Cette synergie ne se produit pas lorsque une combinaison non conforme à l'invention est utilisée, i.e. lorsqu'un sucre est utilisé au lieu d'un polyol et/ou lorsque les polyols ne présentent pas le nombre adéquate d'atomes de carbone.

De plus, les résultats obtenus en valeurs absolues, sont meilleurs lorsqu'une combinaison conforme à l'invention est utilisée, comparativement au tréhalose seul, ou comparativement à d'autres combinaisons de carbohydrates non conformes à l'invention.

Ceci ressort notamment des résultats des essais présentés à l'Exemple 1 ci-après.

La combinaison conforme à l'invention peut être obtenue à partir de composés biosourcés, issus de la technologie des carbohydrates, faciles d'accès et de mise en oeuvre.

La combinaison conforme à l'invention présente par ailleurs l'avantage d'une bonne adéquation entre la quantité que l'on peut solubiliser et la viscosité de la solution résultante. Ceci est particulièrement intéressant dans le domaine des solutions injectables.

Les compositions protéiques comportant la combinaison selon l'invention présentent également l'avantage d'une bonne stabilité chimique, du fait de la faible réactivité des composés employés dans cette combinaison.

Dans la combinaison selon l'invention, le polyol (i) constitué de 12 atomes de carbone peut être un composé unique, préférentiellement le maltitol, ou un mélange de polyols constitués de 12 atomes de carbone. De même, le polyol (ii) constitué de 4 à 6 atomes de carbone peut être un composé unique, ou un mélange de polyols constitué de 4 à 6 atomes de carbone.

Il est ainsi entendu que, dans la présente invention, lorsque l'on se réfère à une composition comprenant la combinaison conforme à l'invention : le polyol (i) inclut tous les polyols constitués de 12 atomes de carbone de la composition, et le polyol (ii) inclut tous les polyols constitués de 4 à 6 atomes de carbone de la composition.

Dans la combinaison selon l'invention, le polyol (i) constitué de 12 atomes de carbone comprend préférentiellement le maltitol, à une teneur préférentiellement supérieure à 50 %, préférentiellement supérieure à 70 %, tout préférentiellement supérieure à 90 % ; ces pourcentages étant exprimés en poids sec de maltitol par rapport au poids sec total des polyols constitués de 12 atomes de carbones. Préférentiellement, le polyol (i) est uniquement composé de maltitol.

Dans la combinaison selon l'invention, le polyol (ii) constitué de 4 à 6 atomes de carbone est préférentiellement constitué de 5 ou 6 atomes de carbone, et est préférentiellement choisi parmi le sorbitol, le mannitol, le xylitol, ou l'un de leurs mélanges. Il est préférentiellement constitué de 6 atomes de carbone, et est préférentiellement choisi parmi le sorbitol, le mannitol, ou un mélange de ceux-ci, par exemple un mélange dans un rapport en poids sec égal à 50/50.

Préférentiellement, le rapport en poids sec entre le polyol (i) et le polyol (ii) de la combinaison selon l'invention varie de 5/95 à 95/5, préférentiellement de 10/90 à 90/10 préférentiellement de 15/95 à 95/15, préférentiellement de 20/80 à 80/20, préférentiellement de 25/75 à 75/25, préférentiellement de 30/70 à 70/30. Ce rapport est par exemple égal à 30/70 ou égal à 70/30. Ce rapport varie tout préférentiellement de 35/65 à 65/35, préférentiellement de 40/60 à 60/40, préférentiellement de 45/55 à 55/45. Il est par exemple égal à 50/50.

Dans la présente invention, le terme « protéine » est au sens large, tel qu'il est habituellement compris de l'homme du métier. Cela couvre en particulier les protéines quel que soit leur mode d'obtention, et quel que soit leur nombre de sousunités. Cela couvre également les fragments de protéines, les peptides et les oligopeptides. Il peut s'agir de protéines natives, de protéines recombinantes, ou de protéines de fusion. Il s'agit préférentiellement de protéines comportant au moins 5 acides aminés, préférentiellement au moins 10 acides aminés, préférentiellement au moins 50 acides aminés, tout préférentiellement au moins 100 acides aminés. Il est entendu que la protéine utile à l'invention, lorsqu'elle est issue de la nature, par exemple d'une plante, est généralement une protéine isolée.

Dans la présente invention, les protéines sont préférentiellement choisies parmi les protéines thérapeutiques ou les protéines destinées à une utilisation industrielle. Ces protéines thérapeutiques peuvent être choisies, de manière non limitative, parmi les enzymes, les cytokines, les hormones, les facteurs de croissances, les facteurs plasmatiques, les vaccins, les anticorps. Des exemples de telles protéines thérapeutiques sont l'érythropoïétine, l'insuline, les anticorps monoclonaux, les hormones de croissance.

Les protéines destinées à une utilisation industrielle sont préférentiellement des enzymes, préférentiellement des β-amylases.

Un premier objet de l'invention concerne l'utilisation d'une combinaison d'un polyol (i) constitué de 12 atomes de carbone et d'un polyol (ii) constitué de 4 à 6 atomes de carbone, notamment telle que définie avant, comme stabilisateur de protéines.

On entend par « stabilisateur de protéines » au sens de la présente invention un agent qui est en particulier capable d'empêcher ou de ralentir la perte d'activité d'une protéine. Cette perte d'activité peut notamment intervenir lorsque la protéine est soumise à des contraintes par exemple physiques et/ou chimiques et/ou mécaniques, par exemple à des variations de température, de pH, d'humidité.

Préférentiellement, l'agent stabilisateur est utilisé pour stabiliser des protéines soumises à des traitements de cryogénisation et/ou de lyophilisation et/ou de stockage, en particulier de stockage en solution, et/ou de traitement thermique, en particulier de chauffage, en particulier de chauffage en solution.

Cette capacité à stabiliser des protéines peut être déterminée par l'homme du métier par mesure de l'activité des protéines soumises à une ou plusieurs contraintes, en présence ou en l'absence (témoin) de l'agent à tester. Cette activité peut être par exemple, de nature enzymatique, et/ou pharmacologique, et/ou antigénique. Pour mesurer cette activité, on peut utiliser toute technique usuelle connue de l'homme du métier, en fonction de la protéine considérée. On peut par exemple procéder selon la méthode décrite dans l'Exemple 1 ci-après.

La présente invention concerne également une composition protéique thérapeutique comprenant une protéine, un polyol (i) constitué de 12 atomes de carbone et un polyol (ii) constitué de 4 à 6 atomes de carbone.

Préférentiellement, les polyols (i) et (ii) sont tels que définis avant, préférentiellement dans un rapport en poids sec tel que défini avant.

Généralement dans cette composition protéique, les protéines représentent de 0,01 à 80,0 % de la matière sèche, préférentiellement de 1,0 à 40,0 %, par exemple de 1,0 à 10,0 %, ou de 10,0 à 30,0 % ; ces pourcentages étant exprimés en poids sec de protéines par rapport au poids total de matière sèche de ladite composition.

Préférentiellement dans cette composition protéique, le rapport entre la quantité de protéines et la combinaison conforme à l'invention varie de 0,5/99,5 à 99,5/0,5, préférentiellement de 0,5/99,5 à 50,0/50,0, préférentiellement encore de 0,5/99,5 à 10,0/90,0, par exemple de 1,0/99,0 à 5,0/95,0 ; ces rapports étant exprimés en poids sec de protéines sur le poids sec de la combinaison conforme à l'invention.

La composition protéique conforme à l'invention peut se présenter sous la forme d'une composition pulvérulente ou d'une composition liquide.

Lorsque la composition protéique est sous forme liquide, elle comprend généralement de 0,02 à 50,00 % de protéines ; ces pourcentages étant exprimés en poids sec de protéines par rapport au poids total de ladite composition protéique liquide.

Préférentiellement, la composition protéique conforme à l'invention est une composition protéique thérapeutique.

On entend par « composition protéique thérapeutique », une composition thérapeutique utilisant des protéines comme principe actif. Il peut s'agir de protéines de même nature ou d'un mélange de protéines thérapeutiques.

Préférentiellement, la composition protéique thérapeutique conforme à l'invention est sous forme liquide, préférentiellement sous la forme d'une solution injectable, ou sous la forme d'une composition pulvérulente pour solution injectable.

Par « composition pulvérulente pour solution injectable » l'homme du métier entend typiquement une composition pulvérulente lyophilisée, destinée à être reconstituée extemporanément à l'aide du solvant approprié.

L'homme du métier sait comment formuler ces solutions injectables, notamment de façon à ce qu'elles répondent aux exigences réglementaires en la matière.

Lorsque la composition protéique thérapeutique est sous forme liquide, en particulier sous la forme d'une solution injectable, elle comprend généralement de 0,01 à 50,00 % de protéines thérapeutiques, préférentiellement de 0,02 à 30,00 %, préférentiellement encore de 0,02 à 25,00 %, par exemple de 0,03 à 5,00 % ; ces pourcentages étant exprimés en poids sec de protéines thérapeutique par rapport au poids total de ladite composition protéique thérapeutique liquide.

Dans un autre mode de réalisation de l'invention, la composition protéique est une composition enzymatique, préférentiellement une composition de β-amylase, en particulier de β-amylase issue du blé (plus communément appelée « WBA » pour « Wheat Beta-Amylase »).

Lorsque cette composition protéique, en particulier de β-amylase, est sous forme liquide, en particulier sous la forme d'une solution aqueuse, elle comprend généralement de 0,1 à 50,0 % de protéines, préférentiellement de 5,0 à 25,0 %, préférentiellement de 10,0 à 25,0 % ; ces pourcentages étant exprimés en poids sec de protéines, par rapport au poids total de la composition liquide.

Les compositions protéiques conformes à l'invention peuvent en outre comprendre tout autre composé bien connu de l'homme du métier pour l'application considérée.

Par exemple, les compositions protéiques thérapeutiques de l'invention peuvent inclure des principes actifs non protéiques et/ou des additifs.

Des exemples d'additifs, en particulier utiles dans le domaine des solutions injectables sont :
- des agents osmotiques, incluant typiquement le glucose et le NaCl ;
- des régulateurs de pH, par exemple des systèmes tampon du type lactate ou gluconate ;
- des stabilisateurs de protéines autres que la combinaison conforme à l'invention, par exemple des acides aminés tels que la lysine et l'arginine, des protéines véhicules, des hydrocolloïdes, des tensio-actifs, des cyclodextrines natives ou modifiées.

Parce que cela n'est pas nécessaire pour obtenir l'effet de stabilisation des protéines recherché dans la présente invention, les compositions protéiques conformes à l'invention comprennent généralement moins de 50,0 % de stabilisateurs de protéines autres que la combinaison conforme à l'invention, en particulier de protéines véhicules, de tréhalose, ou de polysaccharides, préférentiellement moins de 20,0 %, préférentiellement encore moins de 5,0 %, tout préférentiellement moins de 0,1 % ; ce pourcentage étant exprimé en poids sec de stabilisateurs de protéines autres que la combinaison conforme à l'invention par rapport au poids total de matières sèche des compositions protéiques. En particulier, les compositions protéiques conformes à l'invention peuvent être exemptes de stabilisateurs de protéines autres que la combinaison conforme à l'invention.

La présente invention a également pour objet un procédé de préparation d'une composition protéique conforme à l'invention, comprenant le mélange d'une protéine, d'un polyol (i) constitué de 12 atomes de carbone et d'un polyol (ii) constitué de 4 à 6 atomes de carbone.

Préférentiellement, les polyols (i) et (ii) sont tels que définis avant, préférentiellement dans un rapport en poids sec tel que défini avant.

Enfin la présente invention a pour objet un procédé de stabilisation de protéines caractérisé en ce qu'il consiste à ajouter à des protéines une combinaison d'un polyol (i) constitué de 12 atomes de carbone et d'un polyol (ii) constitué de 4 à 6 atomes de carbone.

Préférentiellement, les protéines et polyols (i) et (ii) sont tels que définis avant, préférentiellement dans un rapport en poids sec tel que défini avant.

Les exemples qui suivent permettent de mieux appréhender l'invention, sans toutefois en limiter la portée.

### EXEMPLE

Dans cet exemple, la capacité de différents carbohydrates à stabiliser les protéines a été testée sur une protéine enzymatique, une β-amylase de blé (WBA). Le niveau de dénaturation de la protéine a été évalué par mesure de la perte d'activité enzymatique de la WBA.

Des solutions aqueuses comprenant 0,4 % de WBA et 20 % de carbohydrate ont été préparées ; ces pourcentages étant exprimés en poids sec par rapport au poids total de la solution aqueuse.

Ces solutions ont ensuite été soumises à un traitement thermique à 70°C durant 3 minutes. Ce traitement permet en particulier d'accélérer la dénaturation qui se produit normalement lors d'un stockage en solution sur une plus longue période, et permet donc de mimer les effets d'un tel stockage.

Après traitement thermique, cette solution a été incubée en présence d'amidon, substrat de la WBA. La réaction a été arrêtée par ajout de soude. L'activité enzymatique a été mesurée par un dosage en retour au thiosulfate de sodium. Un pourcentage de perte d'activité a été calculé à partir des mesures d'activité obtenues avant traitement thermique pour chaque essai.

Les résultats obtenus sont présentés dans le Tableau 1.

Afin de faciliter la lecture, la 1^{ère} colonne indique si les essais sont destinés à illustrer l'invention (« IN-X »), s'il s'agit d'essais comparatifs (« CP-X »), ou s'il s'agit d'essais illustrant l'art antérieur (« AA-X »).

Les colonnes 2 à 4 donnent des indications sur les carbohydrates (i) et (ii) utilisés. La 4^{ème} colonne indique le rapport en poids sec entre le carbohydrate (i) et le carbohydrate (ii) utilisés dans les essais.

La 3^{ème} colonne indique la perte d'activité mesurée selon la méthode décrite cidessus. La 4^{ème} colonne indique s'il y a synergie entre les carbohydrates (i) et (ii) utilisés dans chaque essai. Cet effet de synergie a été calculé à partir des valeurs de perte d'activité obtenues lorsque l'on utilisait les carbohydrates seuls, à 20 %. Ces valeurs, pondérées par les proportions en carbohydrates de la combinaison considérée, ont permis de déterminer les valeurs attendues d'un simple effet additif.

Aux valeurs ainsi calculées ont été retranchées les valeurs de perte d'activité réellement obtenues pour la combinaison considérée. Ainsi, un résultat positif dénote une synergie, tandis qu'un résultat égal à 0 indique une absence de synergie, et un résultat négatif, un antagonisme. L'annotation « + » a été utilisée pour désigner les combinaisons pour lesquelles un résultat supérieur à +4 % était obtenu ; l'annotation « ++ » désigne un résultat supérieur à +6 % ; l'annotation « +++ » désigne un résultat supérieur à +10 % ; l'annotation « 0 », désigne un résultat de 0 % (absence de synergie) ; l'annotation « - » désigne un résultat négatif (antagonisme).

Les essais IN-1 à IN-5 montrent qu'une synergie s'opère entre les carbohydrates (i) et (ii) lorsqu'une combinaison de polyols conforme à l'invention est utilisée. Aucune synergie n'a en revanche été observée dans les essais comparatifs et les essais illustrant l'art antérieur. Pour les essais CP-1, CP-2 et CP-4, au contraire, il se produit un antagonisme entre les carbohydrates (i) et (ii).

De plus, en valeurs absolues également, les combinaisons conformes à l'invention sont celles qui présentent les meilleurs résultats : une perte d'activité moindre a été observée en utilisant les combinaisons conformes à l'invention.

**Tableau 1 :**

| | Carbohydrates utilisés | | | Effet | |
|---|---|---|---|---|---|
| | Carbohydrate (i) | Carbohydrate (ii) | Rapport (i)/(ii) | Perte d'activité | Synergie |
| IN-1 | MALTITOL *Polyol en C12* | SORBITOL *Polyol en C6* | 50/50 | 17 % | +++ |
| IN-2 | MALTITOL *Polyol en C12* | MANNITOL *Polyol en C6* | 50/50 | 21 % | ++ |
| IN-3 | MALTITOL *Polyol en C12* | XYLITOL *Polyol en C5* | 70/30 | 20 % | + |
| IN-4 | MALTITOL *Polyol en C12* | XYLITOL *Polyol en C5* | 50/50 | 23 % | +++ |
| IN-5 | MALTITOL *Polyol en C12* | 50 % MANNITOL | 40/60 | 22 % | +++ |
| | | 50 % SORBITOL *Polyol en C6* | | | |
| CP-1 | SORBITOL *Polyol en C6* | XYLITOL *Polyol en C5* | 50/50 | 53 % | - |
| CP-2 | SORBITOL *Polyol en C6* | MANNITOL *Polyol en C6* | 50/50 | 51 % | - |
| CP-3 | MANNITOL *Polyol en C6* | XYLITOL *Polyol en C5* | 50/50 | 49 % | 0 |
| CP-4 | TREHALOSE *Sucre en C12* | MANNITOL *Polyol en C6* | 50/50 | 49 % | - |
| AA-1 | MALTITOL *Polyol en C12* | TREHALOSE *Sucre en C12* | 50/50 | 26 % | 0 |
| AA-2 | TREHALOSE *Sucre en C12* | - | 100 | 36,6 % | Non pertinent |

## Revendications

1. Utilisation d'une combinaison d'un polyol (i) constitué de 12 atomes de carbone et d'un polyol (ii) constitué de 4 à 6 atomes de carbone, comme stabilisateur de protéines.

2. Utilisation selon la revendication 1, pour stabiliser des protéines soumises à des traitements de cryogénisation et/ou de lyophilisation et/ou de stockage, en particulier de stockage en solution, et/ou de traitement thermique, en particulier de chauffage, en particulier de chauffage en solution.

3. Utilisation selon l'une ou l'autre des revendications 1 ou 2, **caractérisée en ce que** ledit polyol (i) comprend le maltitol.

4. Utilisation selon l'une quelconque des revendications 1 à 3, **caractérisée en ce que** ledit polyol (ii) est choisi parmi le sorbitol, le xylitol, le mannitol, ou l'un de leurs mélanges.

5. Utilisation selon l'une quelconque des revendications 1 à 4, **caractérisée en ce que** le rapport en poids sec entre ledit polyol (i) et ledit polyol (ii) de ladite combinaison varie de 5/95 à 95/5.

6. Composition protéique choisie parmi une composition protéique thérapeutique, ou une composition enzymatique, comprenant une protéine, un polyol (i) constitué de 12 atomes de carbone et un polyol (ii) constitué de 4 à 6 atomes de carbone.

7. Composition protéique selon la revendication 6, dans laquelle ledit polyol (i) comprend le maltitol.

8. Composition protéique selon l'une ou l'autre des revendications 6 et 7, dans laquelle ledit polyol (ii) est choisi parmi le sorbitol, le xylitol, le mannitol, ou l'un de leurs mélanges.

9. Procédé de préparation d'une composition telle que définie dans l'une quelconque des revendications 6 à 8, **caractérisé en ce qu'**il comprend le mélange d'une protéine, d'un polyol (i) constitué de 12 atomes de carbone et d'un polyol (ii) constitué de 4 à 6 atomes de carbone.

## Patentansprüche

1. Verwendung einer Kombination aus einem Polyol (i), das aus 12 Kohlenstoffatomen besteht, und einem Polyol (ii), das aus 4 bis 6 Kohlenstoffatomen besteht, als Proteinstabilisator.

2. Verwendung nach Anspruch 1 zum Stabilisieren von Proteinen, die Behandlungen der Kryogenisierung und/oder Lyophilisierung und/oder Lagerung unterzogen werden, insbesondere der Lagerung in Lösung, und/oder der Wärmebehandlung, insbesondere Erhitzung, insbesondere Erhitzung in Lösung.

3. Verwendung nach einem der Ansprüche 1 oder 2, **dadurch gekennzeichnet, dass** das Polyol (i) Maltit umfasst.

4. Verwendung nach einem der Ansprüche 1 bis 3, **dadurch gekennzeichnet, dass** das Polyol (ii) ausgewählt ist aus Sorbit, Xylit, Mannit oder einer ihrer Mischungen.

5. Verwendung nach einem der Ansprüche 1 bis 4, **dadurch gekennzeichnet, dass** das Trockengewichtsverhältnis zwischen dem Polyol (i) und dem Polyol (ii) der Kombination im Bereich von 5/95 bis 95/5 liegt.

6. Proteinzusammensetzung, ausgewählt aus einer therapeutischen Proteinzusammensetzung oder einer Enzymzusammensetzung, die ein Protein, ein Polyol (i), das aus 12 Kohlenstoffatomen besteht, und ein Polyol (ii), das aus 4 bis 6 Kohlenstoffatomen besteht, umfasst.

7. Proteinzusammensetzung nach Anspruch 6, wobei das Polyol (i) Maltit umfasst.

8. Proteinzusammensetzung nach einem der Ansprüche 6 und 7, wobei das Polyol (ii) ausgewählt ist aus Sorbit, Xylit, Mannit oder einer ihrer Mischungen.

9. Verfahren zur Herstellung einer Zusammensetzung wie in einem der Ansprüche 6 bis 8 definiert, **dadurch gekennzeichnet, dass** es das Mischen eines Proteins, eines Polyols (i), das aus 12 Kohlenstoffatomen besteht, und eines Polyols (ii), das aus 4 bis 6 Kohlenstoffatomen besteht, umfasst.

## Claims

1. Use of a combination of a polyol (i) consisting of 12 carbon atoms and of a polyol (ii) consisting of 4 to 6 carbon atoms, as protein stabilizer.

2. Use according to Claim 1, for stabilizing proteins subjected to cryogenization and/or lyophilization treatments and/or storage, in particular storage in solution, and/or heat treatment, in particular by heating, in particular by heating in solution.

3. Use according to either of Claims 1 and 2, **characterized in that** said polyol (i) comprises maltitol.

4. Use according to any one of Claims 1 to 3, **characterized in that** said polyol (ii) is chosen from sorbitol, xylitol, mannitol, or a mixture thereof.

5. Use according to any one of Claims 1 to 4, **characterized in that** the dry weight ratio of said polyol (i) to said polyol (ii) of said combination ranges from 5/95 to 95/5.

6. Protein composition chosen from a therapeutic protein composition or an enzymatic composition, comprising a protein, a polyol (i) consisting of 12 carbon atoms and a polyol (ii) consisting of 4 to 6 carbon atoms.

7. Protein composition according to Claim 6, in which said polyol (i) comprises maltitol.

8. Protein composition according to either of Claims 6 and 7, in which said polyol (ii) is chosen from sorbitol, xylitol, mannitol, or a mixture thereof.

9. Process for preparing a composition as defined in any one of Claims 6 to 8, **characterized in that** it comprises mixing together a protein, a polyol (i) consisting of 12 carbon atoms and a polyol (ii) consisting of 4 to 6 carbon atoms.
